# FASCICULE DE BREVET EUROPEEN

(11) **EP 2 165 698 B1**
(45) Date de publication et mention de la délivrance du brevet: **16.03.2016**
(21) Numéro de dépôt: 08305568.1
(22) Date de dépôt: 18.09.2008
(51) Int. Cl.: A61K 8/64, A61K 8/92, A61Q 19/00, A61K 31/232, A61K 38/06, A61K 38/07, A61P 17/02

(54) **Composition cosmétique destinée au soin de peaux agressées**
Kosmetische Zusammensetzung zur Pflege von angegriffener Haut
Cosmetic composition designed to protect damaged skin

(43) Date de publication de la demande: 24.03.2010
(73) Titulaire: Universkin, 06100 Nice (FR)
(72) Inventeur: Hocquaux, Michel, 75012, Paris (FR); Masson, Claudine, 75014, Paris (FR)
(74) Mandataire: Célanie, Christian

(56) Documents cités:
- WO-A-2008/000974
- FR-A- 2 741 075
- FR-A- 2 834 639
- FR-A- 2 913 603
- US-B1- 6 361 806
- LINTNER, K.: "Biologically active peptides: New perspectives in topical applications" SÖFW JOURNAL, CODEN: SOFJEE; ISSN: 0942-7694, vol. 126, no. 4, 2000, pages 6-10, XP001248937
- DATABASE CA [Online] CHEMICAL ABSTRACTS SERVICE, COLUMBUS, OHIO, US; 12 août 2008 (2008-08-12), PICKART, LOREN: "The human tri -peptide glycine-histidine-lysine and tissue remodeling" XP002519940 extrait de STN Database accession no. 2008:967093 -& JOURNAL OF BIOMATERIALS SCIENCE, POLYMER EDITION , CODEN: JBSEEA; ISSN: 0920-5063, vol. 19, no. 8, 2008, pages 969-988, XP009114015
- DATABASE WPI Week 200732 Thomson Scientific, London, GB; AN 2007-344012 XP002519942 -& WO 2007/037060 A (FANCL CORP) 5 avril 2007 (2007-04-05)
- DATABASE CA [Online] CHEMICAL ABSTRACTS SERVICE, COLUMBUS, OHIO, US; 21 août 2003 (2003-08-21), RODE, JANKO: "Study of autochthon Camelina sativa (L.) Crantz in Slovenia" XP002519941 extrait de STN Database accession no. 2003:650048 & JOURNAL OF HERBS, SPICES & MEDICINAL PLANTS , 9(2/3 AND 4), 313-318 CODEN: JHEPEF; ISSN: 1049-6475, 2002,

## Description

La présente invention a pour objet de nouvelles compositions cosmétiques, dermatologiques ou pharmaceutiques présentant un effet apaisant et restructurant du derme pour lutter contre les agressions liées, et plus particulièrement contre les cicatrices, à une intervention de chirurgie esthétique ou à un traitement de la peau par des appareillages tels que des lasers.

Pour lutter efficacement contre les conséquences de ces interventions, les actifs associés présentent une synergie remarquable alliant un effet apaisant immédiat avec une action restructurante accélérée.

Les femmes ont tendance actuellement à vouloir paraître plus jeune le plus longtemps possible et cherchent à estomper les marques du vieillissement de la peau qui se traduit par un relâchement du tissu cutané et par l'apparition de ridules et de rides.

On traite en général les effets du vieillissement à l'aide de produits cosmétiques contenant des actifs agissant sur la peau.

Depuis quelques années, un véritable engouement s'est manifesté pour des actes interventionnistes à visée esthétique réalisés par des professionnels, tels que la chirurgie réparatrice de la face, du cou, du corps, du cuir chevelu et l'utilisation de différents appareillages tels que les lasers, les lampes flash qui permettent de traiter de nombreuses lésions de la peau (taches vasculaires, taches pigmentaires) ou de lutter contre le vieillissement et les rides.

Les femmes qui s'adressent à ces techniques ont ressenti les premiers effets du vieillissement tels que fragilité et diminution de la souplesse de la peau, apparition des ridules et des rides.

Ces phénomènes sont dus essentiellement à de profondes modifications caractérisées par des altérations de la Matrice Extracellulaire (MEC).

L'épiderme et le derme séparés par la membrane basale, constituent le revêtement cutané qui repose sur l'hypoderme.

L'épiderme constitue la couche la plus superficielle de la peau et en assure sa résistance et son imperméabilité.

Le derme, la couche interne de la peau, tissu conjonctif est composé de cellules (essentiellement fibroblastes) dispersées dans un milieu complexe appelé Matrice Extracellulaire (MEC).

Cette matrice se compose de fibres de collagène et d'élastine, de glycoprotéines (fibronectine et laminine) de protéoglycanes.

La matrice extracellulaire constitue une charpente pour les cellules qui permet la cohésion des tissus et des organes dans les organismes pluricellulaires.

Le derme est le siège de profondes modifications au cours du vieillissement cutané, les changements les plus significatifs de la Matrice Extracellulaire (MEC) surviennent dans le derme.

La fragmentation des fibres de collagène, les altérations du matériel élastique représentent certaines caractéristiques du derme vieillissant.

Le principal composant de la MEC est le collagène, composant protéique majoritaire du derme constitué de 2 sous-unités : le collagène I (80% chez l'adulte) et le collagène III.

Ensemble ils constituent le système fibrillaire qui assure la résistance de la peau.

La fibronectine et la laminine sont des glycoprotéines de structure localisées au niveau de la jonction épidermique ; la fibronectine en tant que constituant essentiel de la Matrice provisoire lors de la réparation cutanée est particulièrement importante dans le processus de cicatrisation.

Les altérations des qualités mécaniques de la peau âgée sont la conséquence de l'atrophie du réseau structural de la MEC du derme et la prévention du vieillissement cutanée passe par le maintien de l'intégrité de cette matrice.

Les conséquences de ces actes interventionnistes (chirurgie réparatrice, traitement lasers, ...) sont l'apparition de phénomènes inflammatoires et la formation de lésions cicatricielles.

Le tri-peptide humain GHK est connu pour certaines actions biologiques au niveau des tissus humains (Loren Pickart, 2008, « The human tri-peptide GHK and tissue remodeling », Journal of Biomaterial Science Polymer Edn, Vol. 19, No. 8, pp. 969-988). De plus une utilisation traditionnelle de l'huile de cameline est connue pour traiter des brûlures et plaies de la peau (Janko Rode, 2002, « Study of autochthon Camelina sativa (L.) Crantz in Slovenia », Journal of Herbs, Spices & Medicinal Plants, Vol. 9 (No. 2/3 and 4), pp. 313-318).

La demanderesse a constaté que l'on pouvait d'une part remédier aux effets délétères de l'inflammation et de la cicatrisation, et d'autre part favoriser la réparation et la restructuration de la MEC en associant :
- un peptide biomimétique (a), tel que défini plus loin, dans une concentration de 10⁻⁸M à 10⁻²M ;
- avec l'huile de caméline dans une concentration comprise entre 1% et 20% par poids.

Dans le cadre de la présente invention, on entend par :
- Gly, la glycine,
- Lys, la lysine,
- His, l'histidine,
- Ac, acétyle

Le peptide (a) selon l'invention est choisi parmi les peptides suivants ou leurs mélanges :
- H-Gly-His-Lys-OH
- H-Gly-His-Lys-NH₂
- Ac-Gly-His-Lys-OH
- Ac- Gly-His-Lys-NH₂.

De façon avantageuse, le peptide (a) répond à la formule H-Gly-His-Lys-OH.

Ces peptides ont été décrits dans les Brevets FR 2 741 075 et FR 2 857 597 déposés par l'Institut Européen de Biologie Cellulaire.

Cette famille de peptides présente le pouvoir de mimer le signal naturel du renouvellement de la peau et la capacité de réactiver la synthèse des protéines de la matrice extracellulaire, tels que les collagènes, la fibronectine, les laminines, l'élastine.

En particulier le peptide de formule H-Gly-His-Lys-OH **(tripeptide-1 :** dénomination INCI) est commercialisé sous la marque Kollaren fabriqué par l'Institut Européen de Biologie Cellulaire

L'huile végétale (b) selon l'invention est l'huile de cameline. La teneur en acide alpha-linoléiques de cette huile est la suivante (en % en poids):
-huile de cameline 35%.

La cameline (Camelina Sativa) est une crucifère de la famille des Brassicaceae. Sa teneur en tocophérols (vitamine E) varie entre 54 et 78 mg/100g. Elle est disponible commercialement, en particulier chez la société PHYTOCOS.

Il est connu que les lipides sont des constituants essentiels de notre organisme.

Ils jouent un rôle important au niveau cutané, d'un point de vue structurel « fonction barrière cutanée », d'un point de vue mécanique « souplesse membranaire » et d'un point de vue biologique « implication dans les phénomènes inflammatoires ».

Certains de ces lipides ne sont pas synthétisés par notre organisme, bien qu'indispensables, tels les acides gras essentiels. Leur absorption déclenche, à l'aide de plusieurs enzymes, une cascade de réactions chimiques.

Dans la famille des AGPI omega-3, seul l'acide alpha-linolénique (ALA) est dit essentiel car l'organisme ne peut le fabriquer.

Il est le précurseur de :
❖ L'acide eicosapentanoïque (EPA) qui lui-même peut se transformer en eicosanoïdes de série-3 qui ont des effets anti-inflammatoires. Cet acide EPA joue un rôle régulateur des processus inflammatoires.
❖ L'acide docosahexanoïque (DHA) qui est capable de stimuler l'expression de la glutathion peroxydase, d'où une protection anti-oxydante.

De nombreuses études ont montré qu'une supplémentation en acides omega-3 DHA et EPA avait un effet sur la peau.

Ces effets très prometteurs sur la peau, obtenus après supplémentation alimentaire, ont conduit la cosmétique à s'intéresser à l'utilisation par voie topique des acides EPA et DHA.

Leurs utilisations s'est avérée limitées par leurs caractéristiques organoleptiques liées à leur origine.

La source principale de ces AGPI est les huiles de poissons riches en EPA/DHA.

Or ces huiles présentent de nombreux motifs de contestation qui ne permettent pas leur utilisation par voie topique :
- Qualité de la Matière Première,
- Reproductibilité de cette Matière Première,
- Stabilité des caractéristiques techniques, particulièrement l'odeur,
- Méthodes d'extraction (désodorisation).

La demanderesse vient de découvrir que l'acide alpha-linolénique peut-être biotransformé par les cellules de la peau que sont les kératinocytes pour produire les deux AGPI oméga-3 que sont les EPA/DHA.

Ce phénomène de métabolisation de l'acide alpha-linolénique en EPA et DHA connu par voie générale n'avait jamais été démontré par voie topique.

Cette biotransformation permettant de générer EPA/DHA par les cellules de la peau joue un rôle important dans les processus inflammatoires.

Elle permet de résoudre les problèmes liés à l'utilisation directe des acides EPA/DHA constituant des huiles de poissons.

Pour que cet effet soit efficace, il est nécessaire d'utiliser des huiles contenant de grandes quantités d'acide alpha-linolénique. Dans le cadre de la présente invention il s'agit de l'huile de cameline.

En outre la demanderesse a constaté que l'on parvenait à atteindre l'efficacité la plus performante en combinant le tripeptide-1, et l'Huile de Cameline, comme décrit plus haut.

La combinaison de ces 2 actifs permet de traiter efficacement tous types de peaux agressés mécaniquement, notamment la zone la plus atteinte comme les cicatrices.

L'huile de cameline, une des huiles les plus riches en acide alpha-linolénique est très vite absorbée par la peau et agit à très court terme. Elle assure rapidement sont action sur le processus inflammatoire, suite à la bioconversion de l'acide alpha-linolénique en EPA/DHA.

De plus, elle a une action sur la barrière cutanée dont elle favorise la reconstitution.

Le tripeptide-1 (INCI), qui mime le signal naturel du renouvellement de la peau, est un actif de choix pour la réparation et la reconstruction de la MEC, en agissant sur des différentes composantes de sa structure.

Son action promotrice de la synthèse du collagène I et II, de fibronectine, de laminine et de l'élastine a été démontrée in vitro.

Son action contribue à la restauration des propriétés biomécaniques de la peau, telles que la fermeté, l'élasticité et favorise la cicatrisation.

La combinaison de ces deux actifs permet d'obtenir une véritable action prolongée d'apaisement et de réparation. Il y a donc une véritable synergie entre ces deux actifs.

La présente invention concerne donc une composition cosmétique, dermatologique ou pharmaceutique caractérisée en ce qu'elle comprend dans un milieu physiologiquement acceptable la combinaison selon la présente invention.

Au sens de la présente invention on entend par milieu physiologiquement acceptable, tout milieu qui est acceptable du point de vue physiologique, c'est-à-dire comprenant un ou plusieurs excipient acceptables du point de vue physiologique et en particulier un ou plusieurs excipients cosmétiquement, pharmaceutiquement ou dermatologiquement acceptables.

En particulier le milieu physiologiquement acceptable est en général à base d'eau ou de solvant, par exemple des alcools, des éthers ou des glycols. Les excipients particulièrement avantageux dans le cadre de la présente invention permettent notamment une pénétration cutanée afin d'améliorer les propriétés et l'accessibilité des principes actifs.

Les compositions selon l'invention peuvent également contenir des agents tensioactifs, des conservateurs, des agents stabilisants, des émulsifiants, des épaississants, d'autres principes actifs conduisant à un effet complémentaire ou éventuellement synergique, des oligo-éléments, des huiles essentielles, des parfums, des colorants, du collagène, des filtres chimiques ou minéraux, des agents hydratants, des eaux végétales, ou des eaux thermales.

La composition cosmétique, dermatologique ou pharmaceutique selon l'invention est avantageusement destinée à une administration par voie topique. Elle pourra donc se présenter sous les formes qui sont habituellement connues pour ce type d'administration, c'est-à-dire notamment les lotions, les mousses, les gels, les dispersions, les sprays, les sérums, les masques, les laits corporels, les pommades, les solutions, les émulsions, les gels, les baumes anhydres ou les crèmes.

La composition selon la présente invention présente une teneur en peptide (a) comprise entre 10⁻⁸M et 10⁻²M, avantageusement entre 10⁻⁴M et 10⁻⁶M.

La composition selon la présente invention présente une teneur en huile de cameline (b) comprise entre 1% et 20% en poids par rapport au poids total de la composition, avantageusement entre 5% et 10%.

La présente invention concerne en outre la composition pharmaceutique ou dermatologique selon la présente invention pour utilisation à titre de médicament.

Avantageusement, le médicament est destiné au soin des peaux agressées et/ou fragilisées, à la cicatrisation des plaies, au traitement des brulures, à la réparation tissulaire cutanée, à apaiser la peau, à favoriser la restructuration et/ou la régénération de la peau et/ou la reconstruction cellulaire et/ou à diminuer les oedèmes et/ou l'inflammation de la peau.

De façon avantageuse, le médicament est appliqué sur la peau fragilisée et/ou agressée par des traumas et/ou des traitements chirurgicaux, des traitements par laser, des dermabrasions et/ou des peelings,

Il a pu être mis en évidence que les combinaisons décrites assuraient une atténuation rapide des inflammations liées à l'agression, qu'elle facilitait la maîtrise des réparations tissulaires et qu'elle augmentait la vitesse de réparation du derme.

Si la composition de l'invention est essentiellement destinée à favoriser la cicatrisation et à traiter les peaux agressées par des traitements cosmétiques ou thérapeutiques de la peau tels que des interventions chirurgicales de la peau, des traitements de la peau par laser, de la dermabrasion ou de peelings, elle est également destinée au traitement curatif et préventif du vieillissement cutané.

La présente invention est illustrée dans les exemples suivants.

### Exemple 1 Etude du tripeptide-1^{®} sur la production de protéines de la MEC par les fibroblastes par immunofluorescence.

▪ **Matériel :** fibroblastes de lignée humaine.
▪ **Protocole :** les fibroblastes sont mis en culture en présence ou en absence du tripeptide-1^{®} à 10⁻⁷M.

▪ Etude microscopique par microscopie confocale.

### ➢ Résultats :

| | **AUGMENTATION %** |
|---|---|
| - Collagène de type I | + 305% |
| - Collagène de type II | + 105% |
| - Fibronectine | + 155% |
| - Laminine | + 85% |
| - Elastine | + 15% |

### Exemple 2 Etude de la biotransformation de l'acide alpha-linolénique par les kératinocytes de la peau.

▪ **Matériel :** kératinocytes de lignée NCTC.
▪ **Protocole : -** les kératinocytes sont mis en contact avec l'huile de cameline pendant 30 mn, à la température de 37°C.
   - Après incubation, l'huile est éliminée, les cellules sont rincées et remises au contact du milieu de culture.
   - Des dosages ont lieu 3^{H}, 5^{H}, 24^{H} (J1), 48^{H} (J2), 72^{H} (J3) sur le milieu de culture (surnageant) et à l'intérieur des cellules après lyse.
▪ Dosage HPLC de l'acide alpha-linolénique et des acides EPA et DHA.

### ➢ Résultats :

a) Dans le surnageant, aucune trace d'acide alpha-linolénique n'est retrouvée des 3^{H}.
   Apparition des acides EPA et DHA après 3^{H} d'incubation.
b) Le dosage HPLC révèle dans les cellules de kératinocytes des quantités importantes d'acide alpha-linolénique dès 3^{H} d'incubation et l'apparition de l'acide EPA à J2.

### Exemple 3 Etude de la biotransformation de l'acide alpha-linolénique par des fibroblastes.

▪ **Matériel :** Fibroblastes de lignée MRC5.
▪ **Protocole :** Identique à celui décrit pour l'exemple 2.
▪ Dosage HPLC de l'acide alpha-linolénique et des acides EPA et DHA.

### ➢ Résultats:

Dans le surnageant, l'acide alpha-linolénique est détecté en plus faible quantité que la quantité de départ.

Apparition des acides EPA et DHA après 3^{H} d'incubation, dans le milieu surnageant.

### Exemple 4 Evaluation de l'effet cicatrisant d'une formulation selon l'exemple 8 contenant le tripeptide-1^{®} et huile de Cameline dans un modèle de peau maintenu en survie.

La formulation utilisée dans cet exemple est celle indiquée dans l'exemple 8.

Pour permettre d'évaluer l'effet cicatrisant, un modèle d'altération des capacités réparatrices de la peau a été réalisé par application d'un dermocorticoïde à la surface de l'épiderme afin de quantifier la stimulation du renouvellement cellulaire.

La stimulation du renouvellement cellulaire a été étudiée par quantification du potentiel mitotique des cellules basales.
▪ **Matériel :** Fragments de peau de plusieurs donneurs différents.
▪ **Protocole :** Les fragments de peau sont mis en survie pendant 4 jours avec application d'un dermocorticoïde de classe II à J0 et J1.
   La formulation a été appliquée tous les jours de J1 à J4.
▪ **Résultat :** Analyse immunohistochimique de la prolifération cellulaire.

| | **%** |
|---|---|
| Peau Témoin | **12** ± 6,2 |
| Peau + dermocorticoïde (peau contrôle) | **7,5** ± 2,5 |
| | NS : p = 0,075 |
| Peau + dermocorticoïde + Formule selon l'exemple 8 | **11,7** ± 3,2 |
| | ≠ **p** = 0,02 |

| | |
|---|---|
| ≠: différence statistiquement significative par rapport au modèle d'altération par dermocorticoïde (test apparié du Student, p<0,05). | |

L'effet cicatrisant a été mis en évidence par obtention d'une restauration statistiquement significative de l'index mitotique de l'épithélium après altération expérimentale de la peau.

### Exemple 5 Evaluation de l'effet hydratant et restructurant d'une formulation contenant le tripeptide-1^{®} et l'huile de Cameline selon l'exemple 8 dans un modèle de peau maintenu en survie.

La formulation utilisée dans cet exemple est celle indiquée dans l'exemple 8.

L'effet hydratant et restructurant a été analysé après réalisation d'un modèle d'altération de la barrière cutanée par le lauryl sulfate de sodium (LSS) responsable d'une perte insensible en eau accompagnée d'altération de l'expression de l'involucrine.

L'évaluation de l'expression de l'involucrine permettra de quantifier la protection apportée par la formulation au niveau de la couche cornée et la stimulation de son métabolisme au niveau des kératinocytes de la peau.
▪ **Matériel :** Fragments de peau de plusieurs donneurs différents.
▪ **Protocole :** Les fragments de peau sont mis en survie pendant 4 jours avec 2 applications topiques de LSS à 1% à J0 et J1.
La formulation a été appliquée tous les jours de J1 à J4.
▪ **Evaluation immunohistochimique de l'expression de l'involucrine.**

| | **Score Intensité** | **Score Topographie** |
|---|---|---|
| Peau témoin | **2,4 ±** 0,8 | **2** ± 0,8 |
| Peau + LSS (peau contrôle) | **1,4** ±1,2 | **1,7** ± 1,5 |
| | * **p** = 0,04 | |
| Peau + LSS + formule selon l'exemple 8 | **3** ± 0,9 | **25** ± 1 |
| | **≠ p** = 0,003 | |

| | | |
|---|---|---|
| * : différence statistiquement significative par rapport à la peau témoin (test apparié du Student, p = < 0,05). ≠ : différence statistiquement significative par rapport à la peau altérée par LSS (test apparié de Student, p < 0,005). | | |

L'effet hydratant et restructurant a été visualisé et quantifié après réalisation du modèle d'altération de la barrière cutanée par le LSS, par la mise en évidence d'une augmentation de l'involucrine au niveau de l'épiderme.

L'augmentation de cette expression témoigne de la protection apportée par le complexe, mais également de la stimulation du métabolisme des cellules de la partie supérieure de l'épithélium.

### Exemple 6 Evaluation de l'effet apaisant d'une formulation contenant le tripeptide-1^{®} et l'huile de Cameline selon l'exemple 8 dans un modèle de peau maintenue en survie.

La formulation utilisée dans cet exemple est celle indiquée dans l'exemple 8.

L'effet apaisant a été analysé après réalisation sur la peau d'un modèle de brûlure expérimentale (application d'un gel agarose à 80°C).

L'activité apaisante a été étudiée en analysant la limitation à la fois de la destruction de l'épiderme (correspondant à l'extension de la brûlure) et de la dilatation vasculaire.

**Matériel :** Fragments de peau de plusieurs donneurs différents.

**Protocole :** Les fragments de peau sont mis en survie pendant 4 jours.

Le modèle expérimental d'altération est obtenu par brûlure après application d'un gel d'agarose à 85°C à J0.

La formulation a été appliquée tous les jours jusqu'à J4.

### ▪ Résultats :

### 1) Analyse histologique des altérations cellulaires au niveau de l'épiderme (% de cellules altérées)

| | **%** |
|---|---|
| Peau + brûlure (peau contrôle) | **24,1** ± 25,1 |
| Peau + brûlure + formule selon l'exemple 8 | **15,8** ± 11,6 |

### 2) Analyse histologique de la dilatation des vaisseaux (surface de vaisseaux dilatés en µm²)

| | **µm²** |
|---|---|
| Peau Témoin | **171,6** ± 38,9 |
| Peau + brûlure (peau contrôle) | **270,8** ± 56,9 |
| | * **p** = 0,00005 |
| Peau + brûlure + formule selon l'exemple 8 | **197,8** ± 78,1 |
| | ≠ **p** = 0,003 |

| | |
|---|---|
| * : différence statistiquement significative par rapport à la peau témoin (test apparié de Student, p < 0,05). ≠ : différence statistiquement significative par rapport à la peau contrôle altérée par brûlure (test apparié de Student, p < 0,05). | |

### 3) Analyse histologique de la dilatation des vaisseaux (% de vaisseaux dilatés)

| | **%** |
|---|---|
| Peau Témoin | **87,6** ± 8,85 |
| Peau + brûlure (peau contrôle) | **99,3** ± 1,2 |
| | * p = 0,0004 |
| Peau + brûlure + formule selon l'exemple 8 | **91,25** ± 8,2 |
| | ≠p = 0,02 |

| | |
|---|---|
| * : différence statistiquement significative par rapport à la peau témoin (test apparié de Student, p < 0,05) ≠ : différence statistiquement significative par rapport à la peau contrôle altérée par brûlure (test apparié de Student, p < 0,05) | |

### 4) Analyse histologique de l'oedème (score)

| | **Score** |
|---|---|
| Peau Témoin | **1,73** ± 0,34 |
| Peau + brûlure (peau contrôle) | **2,26** ± 0,64 |
| | * p = 0,036 |
| Peau + brûlure + formule selon l'exemple 8 | **1,88** ± 0,78 |

| | |
|---|---|
| * : différence statistiquement significative par rapport à la peau témoin (test apparié de Student, p < 0,05). | |

L'effet apaisant a été quantifié de façon significative après réalisation sur la peau d'un modèle de brûlure expérimentale par mise en évidence de la limitation de la dilatation des capillaires de la peau.

La limitation de la destruction de l'épiderme (correspondant à l'extension de la brûlure) a été observée de façon plus modérée et non significative.

### Exemple 7 : Emulsion traitante selon l'invention

| **PHASE HUILEUSE** | **% EN POIDS** |
|---|---|
| - Montanov 68 | 5% |
| - Huile de cameline | 5% |
| - Huile de vaseline | 3% |
| - Isopropyl palmitate | 7% |

| **PHASE AQUEUSE** | |
|---|---|
| - Glycérine | 5% |
| - Sepigel 305 | 0,3% |
| - Conservateur | 0,5% |
| - Tripeptide-1^{®} | 10 ppm |
| - Eau qsp | 100% |

### Exemple 8 : formulation selon l'invention

| **CREME GEL** | **%** |
|---|---|
| **A** | |
| - Dow Corning 9040 | 6% |
| - Huile de cameline | 2% |
| - Regu^{®}-Seb | 3% |
| - Sepigel 305 | 3% |

| **B** | |
|---|---|
| - Tripeptide-1^{®} | 10 ppm |
| - Eau | 82% |

## Revendications

1. Composition cosmétique, dermatologique ou pharmaceutique destinée à une administration par voie topique comprenant dans un milieu physiologiquement acceptable la combinaison
a) d'au moins un peptide choisi parmi les peptides suivants ou leurs mélanges :
• H-Gly-His-Lys-OH,
• H-Gly-His-Lys-NH2,
• Ac-Gly-His-Lys-OH,
• Ac-Gly-His-Lys-NH2
b) de l'huile de cameline,
la teneur en huile de cameline (b) étant comprise entre 1% et 20% en poids par rapport au poids total de la composition et la teneur en peptide (a) étant comprise entre 10⁻⁸M et 10⁻²M.

2. Composition selon la revendication 1 **caractérisée en ce que** le peptide (a) répond à la formule H-Gly-His-Lys-OH

3. Composition selon l'une quelconque des revendications 1 ou 2 **caractérisée en ce que** sa teneur en peptide (a) est comprise entre 10⁻⁴M et 10⁻⁶M.

4. Composition selon l'une quelconque des revendications 1 à 3 **caractérisée en ce que** sa teneur en huile de cameline est comprise entre 5% et 10% en poids par rapport au poids total de la composition.

5. Composition pharmaceutique ou dermatologique selon l'une quelconque des revendications 1 à 4 pour utilisation à titre de médicament.

6. Composition pharmaceutique ou dermatologique selon la revendication 5 **caractérisée en ce que** le médicament est destiné au soin des peaux agressées et/ou fragilisées, à la cicatrisation des plaies, au traitement des brulures, à la réparation tissulaire cutanée, à apaiser la peau, à favoriser la restructuration et/ou la régénération de la peau et/ou la reconstruction cellulaire et/ou à diminuer les oedèmes et/ou l'inflammation de la peau.

7. Composition selon la revendication 6 **caractérisée en ce que** le médicament est appliqué sur la peau fragilisée et/ou agressée par des traumas et/ou des traitements chirurgicaux, des traitements par laser, des dermabrasions et/ou des peelings.

8. Composition selon la revendication 6 **caractérisée en ce que** le médicament est appliqué sur les cicatrices.

9. Composition selon les revendications 1-4 **caractérisée en ce qu'**elle est destinée au traitement curatif et préventif du vieillissement cutané.

## Patentansprüche

1. Kosmetische, dermatologische oder pharmazeutische Zusammensetzung, welche für eine topische Anwendung vorgesehen ist, aufweisend in einem physiologisch akzeptablen Medium die Kombination
a) aus mindestens einem aus den folgenden Peptiden oder ihren Gemischen gewähltes Peptid:
• H-Gly-His-Lys-OH,
• H-Gly-His-Lys-NH2,
• Ac-Gly-His-Lys-OH,
• Ac-Gly-His-Lys-NH2
b) aus Leindotteröl,
wobei der Gehalt an Leindotteröl (b) zwischen 1 und 20 Gewichtsprozent in Bezug auf das Gesamtgewicht der Zusammensetzung und der Gehalt an Peptid (a) zwischen 10⁻⁸M und 10⁻²M liegt.

2. Zusammensetzung nach Anspruch 1, **dadurch gekennzeichnet, dass** das Peptid (a) die Formel H-Gly-His-Lys-OH erfüllt.

3. Zusammensetzung nach einem der Ansprüche 1 oder 2, **dadurch gekennzeichnet, dass** der Gehalt an Peptid (a) zwischen 10⁻⁴M und 10⁻⁶M liegt.

4. Zusammensetzung nach einem der Ansprüche 1 bis 3, **dadurch gekennzeichnet, dass** ihr Gehalt an Leindotteröl zwischen 5 und 10 Gewichtsprozent in Bezug auf das Gesamtgewicht der Zusammensetzung liegt.

5. Pharmazeutische oder dermatologische Zusammensetzung nach einem der Ansprüche 1 bis 4 zur Verwendung als Medikament.

6. Pharmazeutische oder dermatologische Zusammensetzung nach Anspruch 5, **dadurch gekennzeichnet, dass** das Medikament zur Pflege von beanspruchter und/oder empfindlicher Haut, zur Wundheilung, zur Behandlung von Verbrennungen, zur Reparatur von Hautgewebe, zum Beruhigen der Haut, zur Förderung der Restrukturierung und/oder der Regeneration der Haut und/oder des zellularen Neuaufbaus und/oder zur Verringerung von Ödemen und/oder von Entzündungen der Haut vorgesehen ist.

7. Zusammensetzung nach Anspruch 6, **dadurch gekennzeichnet, dass** das Medikament auf der durch Traumata und/oder chirurgische Eingriffe, Laserbehandlungen, Dermabrasionen und/oder Peelings empfindlichen und/oder beanspruchten Haut aufgebracht wird.

8. Zusammensetzung nach Anspruch 6, **dadurch gekennzeichnet, dass** das Medikament auf Narben aufgebracht wird.

9. Zusammensetzung nach den Ansprüchen 1 bis 4, **dadurch gekennzeichnet, dass** sie zur heilenden und vorbeugenden Behandlung der Alterung der Haut vorgesehen ist.

## Claims

1. Cosmetic dermatological or pharmaceutical composition intended to be administered by topical means comprising in a physiological acceptable medium the combination of:
a) at least a peptide selected among the following peptides or their mixture:
H-Gly-His-Lys-OH,
H-Gly-His-Lys-NH2,
Ac-Gly-His-Lys-OH,
Ac-Gly-His-Lys-NH2
b) camelina oil,
the content in camelina oil (b) being comprised between 1 and 20% in weight with respect to the total weight of the composition and the content in peptide (a) being comprised between 10⁻⁸ M and 10⁻² M.

2. Composition according claim 1, **characterised in that** the peptide (a) is H-Gly-His-Lys-OH.

3. Composition according one of claims 1 or 2, **characterised in that** the content in peptide (a) is between 10⁻⁴ M and 10⁻⁶ M.

4. Composition according one of claims 1 to 3, **characterised in that** the content in camelina oil is comprised between 5% and 10% in weight with respect to the total weight of the composition.

5. Pharmaceutical or dermatological composition according one of claims 1 to 4 used as drug.

6. Pharmaceutical or dermatological composition according claim 5, **characterised in that** the drug is intended to care damaged and/or weakened skin, for healing of wounds, for treatment of burs, to repair cutaneous tissue, to calm down the skin, to promote the restructuring and/or the regenration of the skin and/or the cellular reconstruction and/or to reduce the oedema and/or the inflammation of the skin.

7. Composition according claim 6, **characterised in that** the drug is applied on the damaged and/or weakened skin by traumas and/or surgical treatments, laser treatments, dermaabrasion and/or peelings.

8. Composition according claim 6, **characterised in that** the drug is applied on the scars.

9. Composition according claims 1 to 4, **characterised in that** it is intended to be used for curative and preventive treatment of the cutaneous ageing.
